# NEUE EUROPÄISCHE PATENTSCHRIFT

(11) **EP 1 321 129 B2**
(45) Veröffentlichungstag und Bekanntmachung des Hinweises auf die Entscheidung über den Einspruch: **08.10.2008**
(45) Hinweis auf die Patenterteilung: 10.12.2003
(21) Anmeldenummer: 02017443.9
(22) Anmeldetag: 03.08.2002
(51) Int. Cl.: A61Q 5/06

(54) **Versprühbare Haargele**
Sprayable hair gels
Gel pulvérisable pour cheveux

(30) Priorität: 21.12.2001 DE 10163500
(43) Veröffentlichungstag der Anmeldung: 25.06.2003
(73) Patentinhaber: Wella Aktiengesellschaft, 64274 Darmstadt (DE)
(72) Erfinder: Krause, Thomas, Dr., 64297 Darmstadt (DE); Duchscherer, Anja, 65589 Hadamar (DE); Hannich, Manuela, 63329 Egelsbach (DE); Henze, Hildegard, 64297 Darmstadt (DE)

(56) Entgegenhaltungen:
- EP-A- 1 210 932
- EP-A- 1 216 693
- WO-A-97/02006
- WO-A-99/25311
- DE-A- 19 907 715
- US-A- 4 110 291
- Carbopol Aqua SF-1 polymer Technical Data sheet TDS-294, 2000, internet site of Noveon

## Beschreibung

Gegenstand der Erfindung ist ein Haarsprayprodukt mit einer Zusammensetzung zur Haarbehandlung in Gelform mit einem Gehalt an Gelbildnern, ausgewählt aus Copolymeren die aufgebaut sind aus mindestens einer Monomerart die ausgewählt ist aus Acryl- oder Methacrylamidoalkylsulfonsäure oder deren Salzen wobei die Monomerart copolymerisiert ist mit mindestens einen Vinyllactam und Acrylatpolymeren mit verdickender Wirkung, hergestellt aus Acryl- oder Methacrylsäure und mindestens einem Acryl- oder Methacrylsäureester.

Haargele werden eingesetzt, um dem menschlichen Haar Festigung und Halt zu geben. Übliche Haargele enthalten in der Regel eine Kombination aus Gelbildnern und haarfestigenden Polymeren. Die für diese Zwecke üblicherweise verwendeten kosmetischen, haarfestigenden Polymere zeigen in wässrigen, alkoholischen oder wässrig-alkoholischen Medien gute Festigungseigenschaften, die nach der Anwendung mehr oder weniger gut die Haare in Form halten und festigen. Häufig ist damit aber ein unbefriedigender Glanz des Haares verbunden. Es sind zwar eine Reihe von Additiven bekannt, welche in der Lage sind, den Haarglanz zu verbessern, z.B. hydrophobe Stoffe wie flüssige Paraffine, Isoparaffine, Silikonöle oder hydrophile Stoffe wie mehrwertige Alkohole, insbesondere Glycerin oder Propylenglykol. Die bekannten Glanzgeber wirken sich aber häufig nachteilig auf andere, erwünschte Eigenschaften eines Haargels aus. Sie können als Weichmacher für das verwendete festigende Polymer wirken und so die Festigungsleistung herabsetzen, sie können inkompatibel mit der wässrigen Gelbasis sein, sie können die Klarheit und Transparenz des Gels, das rheologische Verhalten und damit die Anwendungseigenschaften beeinträchtigen oder sie sind in ihrer Haarglanz erzeugenden Wirkung noch nicht ausreichend.

Nachteile von Gelformulierungen, die üblicherweise aus Tuben entnommen werden, ist deren schlechte Dosierbarkeit und Verteilbarkeit auf dem Haar und die damit verbundene größere und ungleichmäßige Belastung des Haares. Eine bessere Dosierbarkeit und Verteilbarkeit wird durch Sprühprodukte erreicht. Ein Nachteil von versprühbaren Gelen ist, dass sie entweder ein zu grobes Sprühbild ergeben mit einem hohen Anteil an großen Spraytröpfchen, oder dass eine Sprühpumpe mit sehr hohen Betätigungskräften verwendet werden muß, um ein zufriedenstellendes Sprühbild zu erreichen, oder dass, wenn die Versprühbarkeit gut ist, das Sprühbild zu fein ist mit einem aus gesundheitlichen Gründen unerwünscht hohem Anteil an sehr kleinen, inhalierbaren oder lungengängigen Spraytröpfchen.

Die Aufgabe der vorliegenden Erfindung bestand darin, die oben genannten Nachteile herkömmlicher Haargele zu vermeiden, insbesondere ein gelförmiges Produkt zur Verfügung zu stellen, welches einerseits eine verbesserte Haarfestigung bei optimiertem Haarglanz bewirkt, andererseits gut versprühbar ist und ein optimiertes Sprühbild und eine optimierte Tröpfchengrößenverteilung beim Versprühen aufweist.

Es wurde nun gefunden, dass die Aufgabe gelöst wird durch ein Haarsprayprodukt, bestehend aus einem mit einer sprühpumpe versehenen Behälter und einer verdickten, gelförmigen Zusammensetzung enthaltend mindestens einen Gelbildner (A), welcher ausgewählt ist aus
- Copolymeren, aufgebaut aus mindestens einer Monomerart ausgewählt aus Acryl- oder Methacrylamidoalkylsulfonsäure oder deren Salzen wobei die Monomerart copolymerisiert ist mit mindestens einem Vinyllactam und
- verdickenden Acrylatpolymeren, hergestellt aus Acryloder Methacrylsäure und mindestens einem Acryl- oder Methacrylsäureester, wobei die Acrylatpolymere in 1%iger wässriger Lösung nach pH-Einstellung mit Natriumhydroxid auf pH 7,5 eine Viskosität (20°C, Brookfield RVT, 20 U/min) im Bereich von 2000 bis 8000 mPa s bewirken ausgenommen eine treibgasfreie Sprayzubereitung, bestehend aus einem Spendebehälter mit Sprühventil und einer Zusammensetzung mit Gehalt an 55 Teilen Ethanol, 0,05 Teilen Tocopherolacetat, 0,1 Teilen 1-(2-Ethylhexyloxy)-glycerin, 3,0 Teilen Triethylcitrat, 0,01 Teilen 2,2',4,4'-Tetrahydroxybenzophenon, 0,2 Teilen eines Gemisches aus Polyacrylamid, C13-C14-Isoparaffin und Laureth-7, 1,5 Teilen Acrylates Copolymer, 0,5 Teilen hydriertes Rizinusöl-Oxethylat mit 40 mol Ethylenoxid, 1,2 Teilen Parfümöl und ad 100 Teilen Wasser und NaOH, wobei der pH-Wert 6,51 beträgt (wie beschrieben in EP 1210932 A2).

Bevorzugte Sprühpumpen sind solche, welche eine Betätigungskraft von maximal 40 N, vorzugsweise von 10 bis 30 N, besonders bevorzugt von 12 bis 25 N bei einer Ausbringrate von 0,1 bis 0,3 ml, vorzugsweise von 0,15 bis 0,25 ml pro Hub aufweisen.

Die Viskosität des Gels beträgt vorzugsweise von 100 bis 5000 mPa s, besonders bevorzugt von 200 bis 1000 mPa s, ganz besonders bevorzugt von 250 bis 800 mPa s, gemessen als dynamische Viskositätsmessung mit einem Bohlin Rheometer CS, Messkörper C25 bei einer Temperatur von 25°C und einer Schergeschwindigkeit von 50 s⁻¹.

Der Gelbildner (A) wird vorzugsweise in einer Menge von 0,1 bis 10, besonders bevorzugt von 0,2 bis 8 Gew.% und das haarfestigende Polymer (B) in einer Menge von vorzugsweise 0,1 bis 15, besonders bevorzugt von 0,5 bis 10 Gew.% eingesetzt. Das erfindungsgemäße Haargel bewirkt eine gute Haarfestigung und einen verbesserten Haarglanz bei gleichzeitig verbessertem Sprühverhalten, wenn es mittels einer Sprühvorrichtung versprüht wird.

### Gelbildner(A)

Die erfindungsgemäßen Gelbildner zeichnen sich dadurch aus, dass sie gegenüber herkömmlichen Gelbildnern und Vedickern menschlichen Haaren mehr Glanz und mehr Festigung verleihen. Ein erfindungsgemäß geeigneter Gelbildner ist ein Copolymer, aufgebaut aus mindestens einer Monomerart ausgewählt aus Acryl- oder Methacrylamidoalkylsulfonsäure oder deren Salzen. Geeignete Salze sind solche mit Ammonium-, Alkalimetall- oder Erdalkalimetallkationen. Das Polymer ist vorzugsweise aufgebaut aus Monomeren der allgemeinen Formel H₂C=CH-C(=O)-NH-A-SO₃H oder deren Salzen, wobei A für eine divalente C2- bis C6-, vorzugsweise für eine C3- oder C4-Kohlenwasserstoffgruppe steht, besonders bevorzugt ist die Gruppe -C(CH₃)₂-CH₂-. Dieses Monomer ist copolymersisiert mit mindestens einem Vinyllactam, besonders bevorzugt Vinylpyrrolidon. Ein besonders bevorzugter Gelbildner ist einer mit der INCI-Bezeichnung Ammonium Acryloyldimethyltaurate/VP Copolymer. Ein geeignetes Handelsprodukt ist Aristoflex® AVC.

Weitere, erfindungsgemäß geeignete Gelbildner sind Acrylatpolymere mit verdickender Wirkung. Hierbei handelt es sich um Copolymere aus mindestens einer ersten Monomerart ausgewählt aus Acrylsäure und Methacrylsäure und mindestens einer zweiten Monomerart ausgewählt aus Acrylsäureestern und Methacrylsäureestern, insbesondere den einfachen Estern wie z.B. die C1- bis C4-Alkylester. Diese Polymere haben die INCI-Bezeichnung Acrylates Copolymer. Unter verdickenden Acrylatpolymeren werden solche Polymere verstanden, welche in 1%iger wässriger Lösung nach pH-Einstellung mit Natriumhydroxid auf pH 7,5 eine Viskosität (20°C, Brookfield RVT, 20 U/min) im Bereich von 2000 bis 8000 mPa s, vorzugsweise von 3000 bis 6000 mPa s bewirken. Ein geeignetes Handelsprodukt ist Carbopol® Aqua SF-1.

Die Säuregruppen sind in den eingesetzten Gelbildnern vorzugsweise durch organische oder anorganische Basen ganz oder teilweise, vorzugsweise zu 50 bis 100% neutralisiert. Geeignete Neutralisationsmittel sind primäre oder sekundäre Amine, insbesondere Aminoalkanole mit vorzugsweise 1 bis 10 C-Atomen und 1 bis 3 Hydroxygruppen wie z.B. Aminomethylpropanol (AMP), Triethanolamin, Tetrahydroxypropylethylendiamin oder Monoethanolamin, aber auch Ammoniak, NaOH, KOH u.a..

### Haarfestigendes Polymer (B)

Das haarfestigende Polymer (B) kann nichtionisch, anionisch, kationisch, zwitterionisch oder amphoter sein, ist aber vorzugsweise nichtionisch oder anionisch. Es kann ein synthetisches oder ein natürliches Polymer sein. Unter natürlichen Polymeren werden auch chemisch modifizierte Polymere natürlichen Ursprungs verstanden. Bevorzugt sind insbesondere solche Polymere, die eine ausreichende Löslichkeit in Wasser, Alkohol oder Wasser/Alkohol-Gemischen besitzen, um in dem erfindungsgemäßen Mittel in vollständig gelöster Form vorzuliegen. Unter haarfestigenden Polymeren werden erfindungsgemäß solche Polymere verstanden, die bei Anwendung in 0,01 bis 5%iger wässriger, alkoholischer oder wässrig-alkoholischer Lösung oder Dispersion in der Lage sind, auf dem Haar eine haarfestigende Wirkung zu erzielen.

Geeignete synthetische, nichtionische haarfestigende Polymere sind Homo- oder Copolymere, die aus mindestens einem der folgenden Monomere aufgebaut sind: Vinylpyrrolidon, Vinylcaprolactam, Vinylester wie z.B. Vinylacetat, vinylalkohol, Acrylamid, Methacrylamid, Alkyl- und Dialkylacrylamid, Alkylund Dialkylmethacrylamid, Dialkylaminoalkylmethacrylamid, Dialkylaminoalkylacrylamid, Alkylacrylat, Alkylmethacrylat, Propylenglykol oder Ethylenglykol, wobei die Alkylgruppen dieser Monomere vorzugsweise C1- bis C7-Alkylgruppen, besonders bevorzugt C1- bis C3-Alkylgruppen sind. Geeignet sind z.B. Homopolymere des Vinylcaprolactams, des Vinylpyrrolidons oder des N-Vinylformamids. Weitere geeignete haarfestigende Polymere sind z.B. Copolymerisate aus Vinylpyrrolidon und Vinylacetat, Terpolymere aus Vinylpyrrolidon, Vinylacetat und Vinylpropionat, Terpolymere aus Vinylpyrrolidon, Vinylcaprolactam und Dialkylaminoalkyl(meth)acrylat, Terpolymere aus Vinylpyrrolidon, Vinylcaprolactam und Dialkylaminoalkyl(meth)-acrylamid, Polyacrylamide, Polyvinylalkohole, sowie haarfestigende Polyethylenglykol/Polypropylenglykol Copolymere. Besonders bevorzugte nichtionische Polymere sind Polyvinylpyrrolidon und Polyvinylpyrrolidon/Vinylacetat Copolymere. Bevorzugt sind nichtionische Vinyllactam Homo- oder Copolymere. Geeignete Vinyllactame sind z.B. Vinylcaprolactam und Vinylpyrrolidon. Besonders bevorzugt sind Polyvinylpyrrolidon, Polyvinylcaprolactam und Vinylpyrrolidon/Vinylacetat Copolymere. Bevorzugte Handelsprodukte sind Luviskol® VA 37 und Luviskol® VA 64.

Geeignete anionische haarfestigende Polymere können natürliche oder synthetische Homo- oder Copolymere mit Säuregruppen enthaltenden Monomereinheiten sein, welche gegebenenfalls mit Comonomeren, die keine Säuregruppen enthalten, copolymerisiert sind. Die Säuregruppen sind vorzugsweise ausgewählt aus -COOH, -SO₃H, -OSO₃H, -OPO₂H und -OPO₃H₂, von denen die Carbonsäuregruppen bevorzugt sind. Die Säuregruppen können unneutralisiert, teilweise oder vollständig neutralisiert vorliegen. Sie liegen vorzugsweise zu 50 bis 100% in anionischer bzw. neutralisierter Form vor. Als Neutralisationsmittel können die oben genannten Neutralisationsmittel verwendet werden. Geeignete Monomere sind ungesättigte, radikalisch polymerisierbare Verbindungen, welche mindestens eine Säuregruppe tragen, insbesondere Carboxyvinylmonomere. Geeignete Säuregruppen enthaltende Monomere sind beispielsweise Acrylsäure, Methacrylsäure, Crotonsäure, Maleinsäure bzw. Maleinsäureanhydrid oder deren Monoester, Aldehydocarbonsäuren oder Ketocarbonsäuren.

Nicht mit Säuregruppen substituierte Comonomere sind beispielsweise Acrylamid, Methacrylamid, Alkyl- und Dialkylacrylamid, Alkyl- und Dialkylmethacrylamid, Alkylacrylat, Alkylmethacrylat, Vinylcaprolacton, Vinylpyrrolidon, Vinylester, Vinylalkohol, Propylenglykol oder Ethylenglykol, aminsubstituierte Vinylmonomere wie z.B. Dialkylaminoalkylacrylat, Dialkylaminoalkylmethacrylat, Monoalkylaminoalkylacrylat und Monoalkylaminoalkylmethacrylat, wobei die Alkylgruppen dieser Monomere vorzugsweise C1- bis C7-Alkylgruppen, besonders bevorzugt C1- bis C3-Alkylgruppen sind.

Geeignete anionische Polymere sind insbesondere (von der Komponente (A) verschiedene) Copolymere der Acrylsäure oder Methacrylsäure mit Monomeren ausgewählt aus Acrylsäure- oder Methacrylsäureestern, Acrylamiden, Methacrylamiden und Vinylpyrrolidon, Homopolymere der Crotonsäure sowie Copolymere der Crotonsäure mit Monomeren ausgewählt aus Vinylestern, Acrylsäure- oder Methacrylsäureestern, Acrylamiden und Methacrylamiden. Ein geeignetes natürliches Polymer ist beispielsweise Schellack.

Bevorzugte anionische Polymere sind vernetzte oder unvernetzte Vinylacetat/Crotonsäure Copolymere. Ebenso bevorzugt sind partialveresterte Copolymere zwischen Vinylmethylether und Maleinsäureanhydrid. Weitere geeignete anionische Polymere sind z.B. Terpolymere aus Acrylsäure, Alkylacrylat und N-Alkylacrylamid, insbesondere Acrylsäure/Ethylacrylat/N-t-Butylacrylamid Terpolymere oder Terpolymere aus Vinylacetat, Crotonat und Vinylalkanoat, insbesondere Vinylacetat/Crotonat/Vinylneodecanoat Copolymere.

Geeignete filmbildende amphotere Polymere, sind Polymere, welche neben sauren oder anionischen Gruppen als weitere funktionelle Gruppen basische oder kationische Gruppen, insbesondere primäre, sekundäre, tertiäre oder quaternäre Amingruppen enthalten. Beispiele hierfür sind Copolymere gebildet aus Alkylacrylamid (insbesondere Octylacrylamid), Alkylaminoalkylmethacrylat (insbesondere t-Butylaminoethylmethacrylat) und zwei oder mehr Monomeren ausgewählt aus Acrylsäure, Methacrylsäure oder deren Ester, wobei die Alkylgruppen 1 bis 4 C-Atome enthalten, mindestens eines der Monomere eine Säuregruppe aufweist und wie sie z.B. unter dem Handelsnamen Amphomer® oder Amphomer® LV-71 der Firma NATIONAL STARCH, USA erhältlich sind.

Weitere Beispiele für geeignete haarfestigende Polymere sind Copolymere von Acrylsäure, Methylacrylat und Methacrylamidopropyltrimethylammoniumchlorid (INCI-Bezeichnung: Polyquaternium-47), Copolymere aus Acrylamidopropyltrimethylammoniumchlorid und Acrylaten, Copolymere aus Acrylamid, Acrylamidopropyltrimethylammoniumchlorid, 2-Amidopropylacrylamidsulfonat und Dimethylaminopropylamin (INCI-Bezeichnung: Polyquaternium-43) oder Chitosane. Geeignet sind auch Polymere mit Betaingruppen tragenden Monomeren wie z.B. Copolymere aus Methacryloylethylbetain und zwei oder mehr Monomeren von Acrylsäure oder deren einfachen Estern, bekannt unter der INCI-Bezeichnung Methacryloyl Ethyl Betaine/Acrylates Copolymer.

Das erfindungsgemäße Mittel wird bevorzugt in einem wässrigen, einem alkoholischen oder in einem wässrig-alkoholischen Medium mit vorzugsweise mindestens 10 Gew.%, besonders bevorzugt mindestens 50 Gew.% Wasser und vorzugsweise maximal 40 Gew.% Alkohol konfektioniert. Als Alkohole können insbesondere die für kosmetische Zwecke üblicherweise verwendeten niederen Monoalkohole mit 1 bis 4 C-Atomen wie z.B. Ethanol und Isopropanol enthalten sein. Das erfindungsgemäße Mittel weist vorzugsweise einen pH-Wert von mindestens 6, vorzugsweise von mindestens 6,5 auf. Bei geringeren pH-Werten ist die Neutralisierung der Säuregruppen der Gelbildner nicht ausreichend und die Konsistenz ist zu dünn. Besonders bevorzugt ist der pH-Bereich zwischen 6,0 und 8,0. In einer bevorzugten Ausführungsform enthält das Gel zur weiteren Glanzverbesserung mehrwertige Alkohole, vorzugsweise solche mit 2 bis 5 C-Atomen und mit 2 bis 5 Hydroxygruppen in einer Menge von 0,1 bis 15, bevorzugt von 1 bis 10 Gew.%. Besonders bevorzugt sind Glycerin, Ethylenglykol und Propylenglykol, insbesondere 1,2-Propylenglykol.

Das erfindungsgemäße Mittel kann darüber hinaus die für Haarbehandlungsmittel üblichen Zusatzbestandteile enthalten, z.B. Netzmittel oder Emulgatoren aus den Klassen der nichtionischen, anionischen, kationischen oder amphoteren oberflächenaktiven Tenside, wie ethoxylierte Fettalkohole, Fettalkoholsulfate, Alkylbenzolsulfonate, Alkyltrimethylammoniumsalze, Alkylbetaine, in einer Menge von 0,1 bis 15 Gew.%; Feuchthaltemittel; Parfümöle in einer Menge von 0,1 bis 1 Gew.%; Trübungsmittel, wie z.B. Ethylenglykoldistearat, in einer Menge von etwa 0,2 bis 5,0 Gew.%; Perlglanzmittel, wie z.B. ein Gemisch aus Fettsäuremonoalkylolamid und Ethylenglykoldistearat, in einer Menge von etwa 1,0 bis 10 Gew.%; bakterizide und fungizide Wirkstoffe wie z.B. 2,4,4-Trichlor-2-hydroxydiphenylether oder Methylchlorisothiazolion, in einer Menge von 0,01 bis 1,0 Gew.%; Puffersubstanzen, wie z.B. Natriumcitrat oder Natriumphosphat, in einer Menge von 0,1 bis 1,0 Gew.%; Anfärbestoffe, wie z.B. Fluorescein Natriumsalz, in einer Menge von etwa 0,1 bis 1,0 Gew.%; Pflegestoffe, wie z.B. Betain, Panthenol, Pflanzen- und Kräuterextrakte, Protein- und Seidenhydrolysate, Lanolinderivate, in einer Menge von 0,1 bis 5 Gew.%; physiologisch verträgliche Silikonderivate, wie z.B. flüchtige oder nichtflüchtige Silikonöle oder hochmolekulare Siloxanpolymere in einer Menge von 0,05 bis 20 Gew.%; Lichtschutzmittel, Antioxidantien, Radikalfänger, Antischuppenwirkstoffe, in einer Menge von etwa 0,01 bis 4 Gew.%; Fettalkohole, Glanzgeber, Vitamine, Weichmacher, Kämmbarkeitsverbesserer, rückfettende Agenzien und Entschäumer.

Das erfindungsgemäße Mittel wird vorzugsweise in Form eines klaren, transparenten oder zumindest durchscheinenden Gels eingesetzt, wobei das Gel farblos oder eingefärbt sein kann.

Das erfindungsgemäße Mittel zeichnet sich durch seine besonders positiven Sprüheigenschaften aus, insbesondere wenn es mittels einer mechanisch betriebenen Sprühvorrichtung versprüht wird.

Die erfindungsgemäße Zusammensetzung ist gut versprühbar und gut auf Haaren verteilbar. Sie wirkt nicht belastend auf das Haar und ist daher insbesondere auch für feines Haar geeignet. Als haarfestigendes Mittel bewirkt es eine gute Haltbarkeit der Frisur, ohne dass das Haar verklebt oder belastet wird. Feines Haar bekommt Fülle und Volumen. Als haarfestigendes Gel zeigt das Haarbehandlungsmittel bessere festigende Eigenschaften und insbesondere einen deutlich stärkeren Glanz als herkömmliche Gele auf Carbomer-Basis.

Die nachfolgenden Beispiele sollen den Gegenstand der Erfindung näher erläutern.

### Beispiele

| Beispiel 1: Haargel | | | |
|---|---|---|---|
| | A | B | C |
| Carbopol® Aqua SF1 (Acrylates Copolymer, 30%ig in Wasser) | 3,0 g | - | - |
| Ammonium Acryloyldimethyltaurate/VP Copolymer | - | 0,4 g | - |
| Carbopol® 980 (Polyacrylsäure) | - | - | 0,175 g |
| PVP/VA Copolymer | 2,5 g | 2,5 g | 2,5 g |
| Aminomethylpropanol | 1,425 g | 0,0038g | 0,095 g |
| PPG-1-PEG-9 Lauryl Glycol Ether | 0,2 g | 0,2 g | 0,2 g |
| PEG-40 Hydrogenated Castor Oil | 0,18 g | 0,18 g | 0,18 g |
| Parfüm | 0,2 g | 0,2 g | 0,2 g |
| Ethanol | 31,0 g | 31,0 g | 31,0 g |
| Wasser | Ad 100g | Ad 100g | Ad 100g |

Es wurden drei Haargele vergleichbarer Viskosität unter Verwendung von unterschiedlichen Gelbildnern hergestellt. Die Menge an Gelbildner wurde so gewählt, dass die Gele in etwa die gleiche Viskosität und gleiche Konsistenz aufwiesen. Die Zusammensetzungen wurden hinsichtlich ihrer Wirkungen auf den Haarglanz und auf die Haarfestigung sowie hinsichtlich der Tröpfchengrößenverteilung beim Versprühen verglichen. Gele A und B sind erfindungsgemäß, Gel C ist ein herkömmliches Gel auf Carbomer-Basis.

### Glanz

Zur Glanzmessung wurden Strähnen auf graue PVC-Rollen (Breite 50 mm, Durchmesser 75 mm) aufgespannt und mit Gummibändern fixiert. Die Rollen wurden an einem Stativ so befestigt, dass die Strähnen vom Ansatz zur Spitze vertikal verlaufen. Die Strähnen wurden mit 4 Pumphüben aus einem Abstand von 10-12 cm gleichmäßig besprüht und anschließend bei Raumtemperatur getrocknet. Die Glanzmessung erfolgte, indem ein paralleles Lichtbündel auf die Probenoberfläche gerichtet und die Winkelverteilung des reflektierten und des an oder unter der Oberfläche gestreuten Lichts gemessen wurde. Je höher der Anteil des direkt reflektierten und je niedriger der Anteil des gestreuten Lichts ist, desto höher ist der Glanz. Eine Glanzklassifikation kann durch Messung und Bewertung der Winkelverteilung des von der Oberfläche zurückgeworfenen Lichtes erfolgen. Dabei bedeutet eine schmale Winkelverteilung (geringe Halbwertsbreite HWB) stärkeren Glanz und eine breite Winkelverteilung (größere HWB) geringeren Glanz. Die Messung der Winkelverteilung erfolgt mit einer Digitalkamera, die Daten werden in einen Computer eingelesen und mit einer Bildverarbeitungssoftware (Optimate 5.2) ausgewertet. Es wurden 2 Glanzmessungen pro Strähne und 3 Strähnen pro Zusammensetzung, d.h. insgesamt 6 Messungen pro Muster durchgeführt und der Mittelwert gebildet.

| Ergebnisse der Glanzmessungen: | |
|---|---|
| Unbehandelte Strähnen | HWB = 42° |
| Muster A | HWB = 35° |
| Muster B | HWB = 36° |
| Muster C | HWB = 39° |

Der Austausch des in Muster C eingesetzten Verdickers führt überraschenderweise zu einer Erhöhung des Haarglanzes. Die Messergebnisse korrelieren mit einer visuellen Glanzbeurteilung der Strähnen durch ein in der Beurteilung von Haarqualitäten erfahrenes Expertenpanel.

### Haarfestigung

Der Vergleich der Festigungsleistung erfolgte durch Messung der Bruchkraft. Hierfür wurden standardisierte, ungebleichte Haarsträhnen (100er Zählsträhnen) mit einem Standardshampoo gewaschen, mit Wasser gespült und getrocknet. Pro zu testender Zusammensetzung wurden je drei Strähnen in eine Folientasche gelegt und mit Wasser benetzt. Anschließend wurden insgesamt 90 µl der zu testenden Zusammensetzung (30 µl pro Strähne) aufgetragen. Die Folientaschen werden geschlossen und nach einer Einwirkzeit von 10 Minuten werden alle Strähnen auf einer glatten, nicht saugenden Unterlage im Klimaraum bei 20°C und 65% relativer Luftfeuchte über Nacht getrocknet. Danach wurden für jede Strähne insgesamt 9 Bruchkraftwerte ermittelt (je 3 Messungen am Anfang, in der Mitte und am Ende der Strähne). Die Bruchkraft ist ein Maß für die Haarfestigung.

Die Messungen wurden mit einer Bruchkraft-Meßapparatur durchgeführt, wobei die getrockneten Strähnen einem Dreipunkt-Biegeversuch unterworfen werden. Hierbei wird die Strähne an zwei Punkten aufgelegt (Widerlager) und in der Mitte durch einen Kraftsensor belastet. Ein Computer zeichnet die Biegekraft als Funktion der durchgebogenen Strecke in Form eines Diagramms auf. Je größer die maximale Biegekraft (=Bruchkraft), desto größer ist die festigende Wirkung des Produktes. Die maximale Biegekraft wird Bruchkraft genannt, da an diesem Punkt die Festigung gebenden Polymerverbindungen (z.B. Polymerfilm) zwischen den Haaren brechen. Es wurden 3 Messungen pro Strähne und 3 Strähnen pro Zusammensetzung, d.h. insgesamt 9 Messungen pro Muster durchgeführt und der Mittelwert gebildet.

| Ergebnisse der Bruchkraftmessungen: | |
|---|---|
| Muster A | 0,30 N |
| Muster B | 0,26 N |
| Muster C | 0,19 N |

Der Austausch des Verdickers führt überraschend zu einer höheren Haarfestigung.

### Tröpfchengrößenverteilung

Zur Messung der inhalierbaren Anteile mit einem Durchmesser kleiner 10 µm beim Versprühen der Zusammensetzungen, wurden die Zusammensetzunen mit einer Sprühpumpe (Coster GMSP 38/200, Düse V 06.233) versprüht. Die Tröpfchengrößenverteilung wurde mit Hilfe eines Partikelmessgerätes auf Basis von Laserstrahlbeugung (Malvern Particle Sizer) gemessen.

| Anteil an Spraytröpfchen mit einem Durchmesser <10 µm: | |
|---|---|
| Muster A | 0,65 % |
| Muster B | 0,7 % |
| Muster C | 1,0 % |

## Patentansprüche

1. Haarsprayprodukt, bestehend aus einem mit einer Sprühpumpe versehenen Behälter und einer verdickten, gelförmigen Zusammensetzung enthaltend mindestens einen Gelbildner ausgewählt aus
- Copolymeren, aufgebaut aus mindestens einer Monomerart die ausgewählt ist aus Acryl- oder Methacrylamidoalkylsulfonsäure oder deren Salzen und wobei die Momomerart copolymerisiert ist mit mindestens einem Vinyllactam; und
- verdickenden Acrylatpolymeren, die hergestellt sind aus Acryl- oder Methacrylsäure und mindestens einem Acryl- oder Methacrylsäureester und ausgewählt sind aus Acrylatpolymeren, die in 1% iger wässriger Lösung nach pH-Einstellung mit Natriumhydroxid auf pH 7,5 eine Viskosität (20°C, Brookfield RVT, 20 U/min) im Bereich von 2000 bis 8000 mPa s bewirken;
ausgenommen eine treibgasfreie Sprayzubereitung, bestehend aus einem Spendebehälter mit Sprühventil und einer Zusammensetzung mit Gehalt an 55 Teilen Ethanol, 0,05 Teilen Tocopherolacetat, 0,1 Teilen 1-(2-Ethylhexyloxy)-glycerin, 3,0 Teilen Triethylcitrat, 0,01 Teilen 2,2',4,4'-Tetrahydroxybenzophenon, 0,2 Teilen eines Gemisches aus Polyacrylamid, C13-C14-Isoparaffin und Laureth-7, 1,5 Teilen Acrylates Copolymer, 0,5 Teilen hydriertes Rizinusöl-Oxethylat mit 40 mol Ethylenoxid, 1,2 Teilen Parfümöl und ad 100 Teilen Wasser und NaOH, wobei der pH-Wert 6,51 beträgt.

2. Haarsprayprodukt nach Anspruch 1, **dadurch gekennzeichnet, dass** die Sprühpumpe eine mechanisch zu betätigende Pumpe ist, welche eine Betätigungskraft von 10 bis 40 N bei einer Ausbringrate von 0,1 bis 0,3 ml pro Hub aufweist.

3. Haarsprayprodukt nach einem der vorhergehenden Ansprüche, **dadurch gekennzeichnet, dass** die gelförmige Zusammensetzung zusätzlich mindestens ein haarfestigendes Polymer enthält.

4. Haarsprayprodukt nach einem der vorhergehenden Ansprüche, **dadurch gekennzeichnet, dass** die Viskosität der gelförmigen Zusammensetzung von 100 bis 5000 mPa s (gemessen mit einem Bohlin Rheometer CS, Messkörper C25 bei 25°C und einer Schergeschwindigkeit von 50 s⁻¹) beträgt.

5. Haarsprayprodukt nach einem der vorhergehenden Ansprüche, **dadurch gekennzeichnet, dass** die Gelbildner in einer Menge von 0,1 bis 10 Gew.% und die haarfestigenden Polymere in einer Menge von 0,1 bis 15 Gew.% vorliegen.

6. Haarsprayprodukt nach einem der vorhergehenden Ansprüche, **dadurch gekennzeichnet, dass** der polymere Gelbildner ausgewählt ist aus Copolymeren, welche aufgebaut sind aus mindestens einer Monomerart die ausgewählt ist aus Monomeren der allgemeinen Formel H₂C=CH-C(=O)-NH-A-SO₃H oder deren Salzen, wobei A für eine divalente C2- bis C6-Kohlenwasserstoffgruppe steht und wobei die Momomerart copolymerisiert ist mit mindestens einem Vinyllactam.

7. Haarsprayprodukt nach Anspruch 6, **dadurch gekennzeichnet, dass** der polymere Gelbildner ein Ammonium Acryloyldimethyltaurat/Vinylpyrrolidon Copolymer ist.

8. Haarsprayprodukt nach einem der vorhergehenden Ansprüche, **dadurch gekennzeichnet, dass** das haarfestigende Polymer ausgewählt ist aus nichtionischen Vinyllactam Homo- oder Copolymeren.

9. Verwendung eines Polymers, ausgewählt aus
- Copolymeren, aufgebaut aus mindestens einer Monomerart die ausgewählt ist aus Acryl- oder Methacrylamidoalkylsulfonsäure oder deren Salzen und wobei die Momomerart copolymerisiert ist mit mindestens einem Vinyllactam; und
- verdickenden Acrylatpolymeren, hergestellt aus Acryl- oder Methacrylsäure und mindestens einem Acryl- oder Methacrylsäureester und ausgewählt aus Acrylatpolymeren, die in 1%iger wässriger Lösung nach pH-Einstellung mit Natriumhydroxid auf pH 7,5 eine Viskosität (20°C, Brookfield RVT, 20 U/min) im Bereich von 2000 bis 8000 mPa s bewirken
zur Erhöhung des Glanzes von Haaren.

## Claims

1. Hairspray product consisting of a container provided with a spray pump, and a thickened, gel-like composition comprising at least one gel former chosen from
- copolymers constructed from at least one type of monomer which is chosen from acryl- or methacrylamidoalkylsulphonic acid or salts thereof and where the type of monomer is copolymerized with at least one vinyllactam; and
- thickening acrylate polymers which are prepared from acrylic or methacrylic acid and at least one acrylic or methacrylic ester and are chosen from acrylate polymers which, in 1% strength aqueous solution following pH adjustment with sodium hydroxide to pH 7.5, effect a viscosity (20°C, Brookfield RVT, 20 rpm) in the range from 2000 to 8000 mPa s;
with the exception of a propellant-gas-free spray preparation consisting of a dispensing container with spray valve and a composition containing 55 parts of ethanol, 0.05 parts of tocopherol acetate, 0.1 parts of 1-(2-ethylhexyloxy)glycerol, 3.0 parts of triethyl citrate, 0.01 parts of 2,2',4,4'-tetrahydroxybenzophenone, 0.2 parts of a mixture of polyacrylamide, C13-C14-isoparaffin and laureth-7, 1.5 parts of acrylates copolymer, 0.5 parts of hydrogenated castor oil oxethylate with 40 mol of ethylene oxide, 1.2 parts of perfume oil and ad 100 parts of water and NAOH, where the pH is 6.51.

2. Hairspray product according to Claim 1, **characterized in that** the spray pump is a pump which is operated mechanically and which has an operating force of from 10 to 40 N at a dispensing rate of from 0.1 to 0.3 ml per stroke.

3. Hairspray product according to one of the preceding claims, **characterized in that** the gel-like composition additionally comprises at least one hair-setting polymer.

4. Hairspray product according to one of the preceding claims, **characterized in that** the viscosity of the gel-like composition is from 100 to 5000 mPa s (measured using a Bohlin CS rheometer, bob C25 at 25°C and a shear velocity of 50 s⁻¹).

5. Hairspray product according to one of the preceding claims, **characterized in that** the gel formers are present in an amount of from 0.1 to 10% by weight and the hair-setting polymers are present in an amount of from 0.1 to 15% by weight.

6. Hairspray product according to one of the preceding claims, **characterized in that** the polymeric gel former is chosen from copolymers which are constructed from at least one type of monomer which is chosen from monomers of the general formula H₂C=CH-C(=O)-NH-A-SO₃H or salts thereof, where A is a divalent C2- to C6-hydrocarbon group and where the type of monomer is copolymerized with at least one vinyllactam.

7. Hairspray product according to Claim 6, **characterized in that** the polymeric gel former is an ammonium acryloyldimethyltaurate/ vinylpyrrolidone copolymer.

8. Hairspray product according to one of the preceding claims, **characterized in that** the hair-setting polymer is chosen from nonionic vinyllactam homopolymers or copolymers.

9. Use of a polymer chosen from
- copolymers constructed from at least one type of monomer which is chosen from acryl- or methacrylamidoalkylsulphonic acid or salts thereof and where the type of monomer is copolymerized with at least one vinyllactam; and
- thickening acrylate polymers prepared from acrylic or methacrylic acid and at least one acrylic or methacrylic ester and chosen from acrylate polymers which, in 1% strength aqueous solution following pH adjustment with sodium hydroxide to pH 7.5, effect a viscosity (20°C, Brookfield RVT, 20 rpm) in the range from 2000 to 8000 mpa s
for increasing the sheen of hair.

## Revendications

1. Laque pour cheveux, constituée d'un contenant muni d'une pompe à pulvérisation et d'une composition épaissie, sous forme de gel, contenant au moins un agent gélifiant choisi parmi
- des copolymères constitués d'au moins un type de monomère choisi parmi les acides acryl- et méthacrylamidoalkylsulfoniques et leurs sels et le type de monomère étant copolymérisé avec au moins un vinyl-lactame ; et
- des polymères d'acrylate épaississants qui sont préparés à partir d'acide acrylique ou méthacrylique et d'au moins un ester d'acide acrylique ou méthacrylique et sont choisis parmi les polymères d'acrylate qui, en solution aqueuse à 1 %, après ajustement du pH à pH 7,5 à l'aide d'hydroxyde de sodium, procurent une viscosité (à 20°C, RVT Brookfield, 20 tours/min) dans la plage allant de 2 000 à 8 000 mPa.s ;
à l'exception d'une préparation pulvérisable sans gaz propulseur, constituée d'un contenant distributeur à valve de pulvérisation et d'une composition contenant 55 parties d'éthanol, 0,05 partie d'acétate de tocophérol, 0,1 partie de 1-(2-éthylhexyloxy)-glycérol, 3,0 parties de citrate de triéthyle, 0,01 partie de 2,2',4,4'-tétrahydroxybenzophénone, 0,2 partie d'un mélange de polyacrylamide, isoparaffine en C₁₃-C₁₄ et Laureth-7, 1,5 partie de copolymère d'acrylate, 0,5 partie de produit d'oxyéthylation d'huile de ricin hydrogénée avec 40 moles d'oxyde d'éthylène, 1,2 partie d'huile essentielle et, en complément à 100 parties, de l'eau et NaOH, le pH étant égal à 6,51.

2. Laque pour cheveux selon la revendication 1, **caractérisée en ce que** la pompe à pulvérisation est une pompe à actionner mécaniquement qui présente une force d'actionnement de 10 à 40 N à un taux d'application de 0,1 à 0,3 ml par élévation.

3. Laque pour cheveux selon l'une quelconque des revendications précédentes, **caractérisée en ce que** la composition sous forme de gel contient en outre au moins un polymère fixant les cheveux.

4. Laque pour cheveux selon l'une quelconque des revendications précédentes, **caractérisée en ce que** la viscosité de la composition sous forme de gel va de 100 à 5 000 mPa.s (mesurée avec un rhéomètre Bohlin CS, corps de mesure C25 à 25°C et à une vitesse de cisaillement de 50.s⁻¹).

5. Laque pour cheveux selon l'une quelconque des revendications précédentes, **caractérisée en ce que** les agents gélifiants sont présents en une quantité de 0,1 à 10 % en poids et les polymères fixant les cheveux sont présents en une quantité de 0,1 à 15 % en poids.

6. Laque pour cheveux selon l'une quelconque des revendications précédentes, **caractérisée en ce que** l'agent gélifiant polymère est choisi parmi des copolymères qui sont constitués d'au moins un type de monomère choisi parmi les monomères de formule générale H₂C=CH-C(=O)-NH-A-SO₂H et leurs sels, A représentant un groupe hydrocarboné divalent en C₂ à C₆ et le type de monomère étant copolymérisé avec au moins un vinyl-lactame

7. Laque pour cheveux selon la revendication 6, **caractérisée en ce que** l'agent gélifiant polymère est un copolymère acryloyldiméthyltaurate d'ammonium/vinylpyrrolidone.

8. Laque pour cheveux selon l'une quelconque des revendications précédentes, **caractérisée en ce que** le polymère fixant les cheveux est choisi parmi des homo- et copolymères de vinyl-lactame non ioniques.

9. Utilisation d'un polymère choisi parmi
- des copolymères constitués d'au moins un type de monomère choisi parmi les acides acryl- et méthacrylamidoalkylsulfoniques et leurs sels et le type de monomère étant copolymérisé avec au moins un vinyl-lactame ; et
- des polymères d'acrylate épaississants qui sont préparés à partir d'acide acrylique ou méthacrylique et d'au moins un ester d'acide acrylique ou méthacrylique et sont choisis parmi les polymères d'acrylate qui, en solution aqueuse à 1 %, après ajustement du pH à pH 7,5 à l'aide d'hydroxyde de sodium, procurent une viscosité (à 20°C, RVT Brookfield, 20 tours/min) dans la plage allant de 2 000 à 8 000 mPa.s ;
pour accentuer le brillant des cheveux.
